# EUROPEAN PATENT SPECIFICATION

(11) **EP 3 378 446 B1**
(45) Date of publication and mention of the grant of the patent: **04.11.2020**
(21) Application number: 16865821.9
(22) Date of filing: 18.11.2016
(51) Int. Cl.: A61F 2/60, A61F 2/68, A61H 3/00, A61H 1/02, B25J 9/00

(54) **SYSTEM FOR ASSISTING WALKING**
SYSTEM ZUR UNTERSTÜTZUNG DES GEHENS
SYSTÈME D'AIDE À LA MARCHE

(30) Priority: 19.11.2015 ES 201531671
(43) Date of publication of application: 26.09.2018
(73) Proprietor: Tecnimusa, S.L, 30320 Fuente Álamo (Murcia) (ES)
(72) Inventor: GARCÍA LEGAZ, Juan, 30320 Fuente Álamo (Murcia) (ES); BEJARANO HERRUZO, Bartolomé., 30320 Fuente Álamo (Murcia) (ES)
(74) Representative: Díaz Pacheco, Maria Desamparados
(86) International application number: PCT/ES2016/000126
(87) International publication number: WO 2017/085338

(56) References cited:
- EP-A1- 2 754 538
- EP-A2- 2 942 044
- WO-A1-2015/168788
- ES-T3- 2 418 441
- GB-A- 2 301 776
- US-A1- 2012 172 770
- US-A1- 2014 100 493
- US-A1- 2015 164 732
- US-B1- 7 628 766

## Description

### TECHNICAL FIELD OF THE INVENTION

This invention relates to a system that allows a suitable gait in patients who are unable to walk without help.

### BACKGROUND OF THE INVENTION OR STATE OF THE ART

A paraplegia or paraparesis denotes a complete or partial weakness of both lower limbs, having no functional ability to stand up or move around.

Paraplegia or paraparesis is caused by a condition of the spinal cord (generally in the thoracic or lumbar region), peripheral nerves, or muscles (myopathies). Peripheral neuropathies usually present with a pattern of distal weakness, sensory loss, muscle atrophy, and an absence of reflexes. In contrast, spinal paraplegia causes spasticity and a dramatic increase in reflexes and in the level of sensory loss. Injuries to the conus medullaris or cauda equina can lead to a complex set of symptoms which indicate both spinal and peripheral nerve involvement. There are brain disorders that may cause paraplegia/paraparesia which may even affect upper limbs as well, such as brainstem injuries, parasagittal cerebral injuries (meningiomas), or anterior cerebral artery ischemia.

Spinal paraplegia usually occurs as a result of a trauma causing a serious spinal injury with compression or sectioning of the spinal cord, normally by bone fragments from a vertebral fracture or the associated hematoma. Most of the time, the symptoms are acute as the result of an injury, where half of such injuries occur in traffic accidents. In the absence of trauma, spinal cord compression and myelitis are the main causes of acute-onset or rapidly-progressive paraplegia.

The injuries may be of varying degrees, depending on the severity and the location of the injury. The patient may initially experience spinal shock, which produces a loss of or decrease in sensitivity, muscle movement, and reflexes. As inflammation decreases, other symptoms may appear, depending on the location of the injury. The symptoms are generally more serious the higher the region of the spinal cord where the injury occurs. Some of the main symptoms which indicate a serious spinal cord injury are: muscle weakness, loss of voluntary movements of the muscles involved, loss of sensitivity in the lower limbs, loss of bladder and intestinal control, breathing problems, etc.

There are proposals in the state of the art that are based on electromechanical devices designed for patients of this type. They are exoskeletons to be worn on the body developed for medical, military, and civil purposes. There are currently a number of enterprises and research centers they are still trying to commercially develop these exoskeletons, but they have encountered several obstacles, primarily marketing costs and operating times. The enterprises that are closest to launching mass production of these exoskeletons are Honda and Cyberdyne.

Honda has two exoskeleton prototypes to aid in walking; one is a machine design called "BODYWEIGHT SUPPORT ASSIST," which was designed to reduce muscle load in the legs and joints (hip, knees, and ankles). This equipment was created for rehabilitation of the elderly and the disabled.

The enterprise's other prototype is called "WALKING ASSIST DEVICE." It is an exoskeleton which speeds up a person's natural walk by incorporating mechanical force in his or her walking. Embedded sensors allow reading hip mobility intention in order to help the user maintain an upright posture, and the device uses motors to increase the force in the steps. It is intended for elderly persons and for those who have weak leg muscles.

In turn, Cyberdyne has the robotic suit called "HAL." Its applications include: rehabilitation, physical training in the field of medicine, support for people with a disability, use in heavy work or in rescue activities at disaster sites. Unlike those mentioned above, this exoskeleton is for upper and lower limbs. The "HAL" exoskeleton uses sensors attached to skin of the person wearing it to read electrical nerve impulses. According to the signals that are obtained, the unit is controlled to move the joint at the same time it moves the muscles of the person wearing it. If there are no signals, "HAL" also has an autonomous control system that provides movement to the exoskeleton that is similar to human movements. It is necessary to use crutches.

The Human Engineering Laboratory at the University of California, Berkeley (United States) has conducted research on the fundamentals of human power, providing a number of applicable results, including the following lower limb exoskeletons: Bleex, Exohiker, Exoclimber, Hulc, and exoskeletons for medicine; all of these exoskeletons with the exception of the latter ones were designed for military applications. Their designs focus on increasing strength and resistance of soldiers on the battlefield.

The following prior art is briefly described:
- "EXOHIKER": this was the first robotic exoskeleton created by the Berkeley Bionics team and the University of California. It was designed for carrying heavy backpacks for long-distance missions with minor changes in altitude.
- "EXOCLIMBER": this is a robotic exoskeleton that is designed to allow rapidly going up stairs and steep slopes while providing the same transport capacity as ExoHiker.
- XOS: the full-body robotic exoskeleton for military purposes presented by Raytheon Sarcos was Sarcos XOS, which sought to increase the strength and resistance of soldiers. It is used for loading tasks for explosive elements such as missiles. It allows the person wearing it to lift a weight of 100 kg as if it were just 1 kg. Furthermore, it is flexible and allows running or jumping very easily, but this exoskeleton requires an external power supply. The new XOS 2 is a robotic structure operated by a more resistant high-voltage hydraulic system that consumes half the energy, but its main innovation is that it has greater autonomy because it does not require a cable connecting the suit to a power source.
- "REWALK": this is an electronic exoskeleton capable of helping paraplegics to walk and is produced by Argo Medical Technologies. Although the patient needs crutches to keep his or her balance, the system has a remote control that is fitted to the wearer's wrist and puts a series of body sensors into operation, and as a result of the these sensors the legs are motor-powered, which allows selecting the type of movement. Through the remote control, patients can choose to sit up, walk, and go up or down the stairs.
- "BLEEX": is a lower extremity robotic exoskeleton. It is made up of two powered anthropomorphic legs, a power unit, and a backpack-like frame on which a variety of loads can be loaded. This system provides the user with the capacity to carry significant loads with minimal effort over any type of terrain. It allows a person to comfortably squat, bend, swing from side to side, twist, and walk on ascending and descending slopes, and also step over and under obstructions.

Document WO2015/168788A1 discloses an exoskeleton having actuators for performing flexion-extension and abduction-adduction movements of a person's ankle, knee and in particular hip.

### BRIEF DESCRIPTION OF THE INVENTION

In view of the prior art, most lower extremity exoskeletons have been designed for healthy individuals, fundamentally intended for military uses, and exoskeletons for aiding the disabled require a second person or crutches and have pre-programmed movements that prevent autonomy of the individual.

In the military, exoskeletons (pneumatic, hydraulic, etc.) have a programmed accompanying performance to ease the weight carried by the user or to provide the user with greater resistance; however, they lack movement autonomy, so movement must be generated by the extremities of the individual himself or herself.

In contrast, the present invention proposes an exoskeleton to return motor capacity to the individual. To that end, joints such as the ankle are reinforced, where not only the movement of the Achilles tendon (flexion and extension) is motor-powered, but so is the lateral movement of the foot (abduction and adduction). With respect to the existing exoskeletons designed for paraplegics, it can be seen that they do not contemplate movement (abduction and adduction) of the ankle.

In contrast, the present proposal considers this essential function for thorough control of mobility. In all cases, the exoskeleton object of the present invention implements these two movements of the ankle.

In turn, the existing exoskeletons generally only have automated flexion-extension movement in the hip, where the abduction-adduction movements and external-internal rotation are allowed only in military models, and are fixed for paraplegic models. It has been found that all three movements are required for a correct gait; therefore, the present invention implements these three motor-driven movements.

The sensory transmission proposed by the present invention is based on translating flexion of a joint of the upper limb into an actuation in the exoskeleton corresponding to another joint of the lower limb suffering paralysis.

Hereinafter, for the sake of clarity in the description, it must be understood without loss of generality that the term "flexion" refers to the movement of the joint, whether it is extension or flexion, abduction or adduction.

Additionally, an outsole for the foot will be able to transmit the pressure regions to other sensory regions of the body, such as the hand, by means of a glove.

The present invention is directed to a system to assist a person in walking according to claim 1. Subsidiary aspects of the invention are provided in the dependent claims.

### BRIEF DESCRIPTION OF THE DRAWINGS

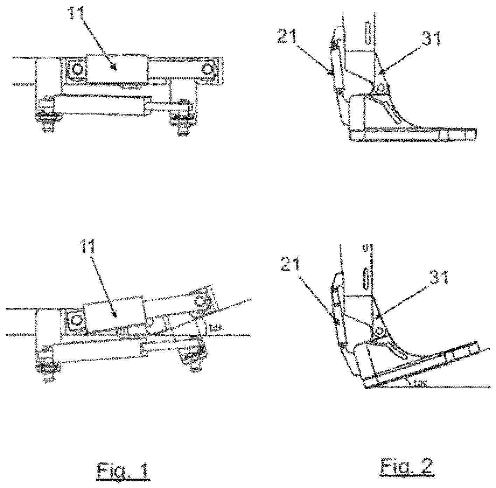

Figure 1 shows an exoskeleton module with a wrist sensor illustrated in a non-flexed (or non-abducted) state and in a flexed state of 10°.
Figure 2 shows an exoskeleton module with an ankle actuator in a non-flexed and a flexed state.

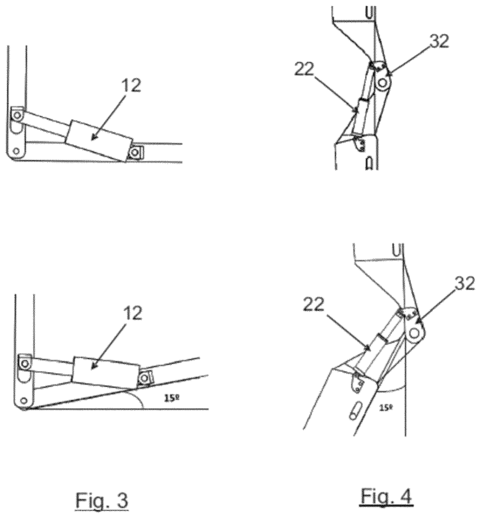

Figure 3 shows an exoskeleton module with an elbow sensor illustrated in a non-flexed state and in a flexed state of 15°.
Figure 4 shows an exoskeleton module with a knee actuator in a non-flexed and a flexed state.

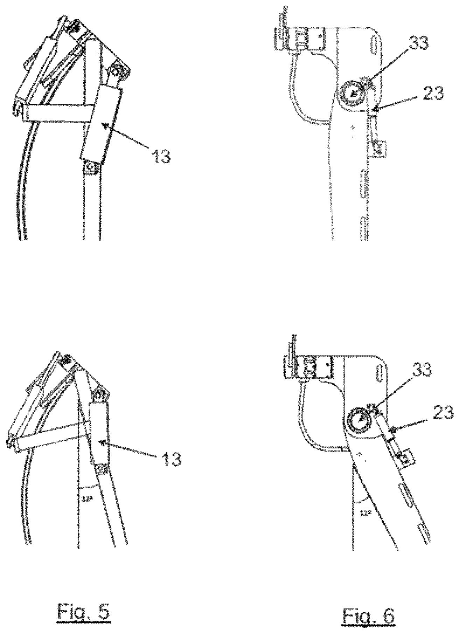

Figure 5 shows an exoskeleton module with a shoulder sensor illustrated in a non-flexed state and in a flexed state of 12°.
Figure 6 shows an exoskeleton module with a hip actuator in a non-flexed and a flexed state.

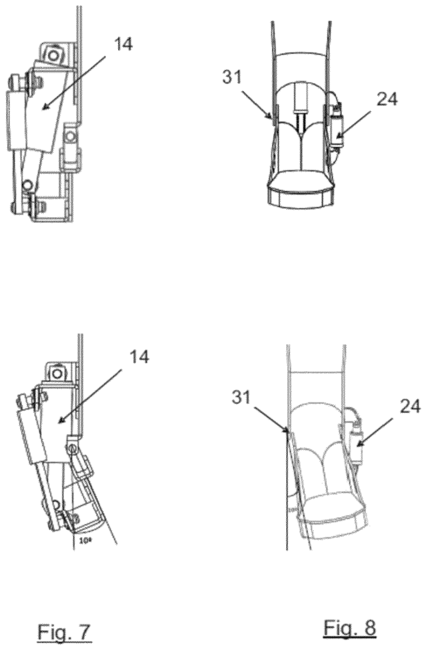

Figure 7 shows an exoskeleton module with a wrist sensor illustrated in a non-abducted-adducted state and in an abducted-adducted state of 10°.
Figure 8 shows an exoskeleton module with ankle actuator in an abducted and a non-abducted state.

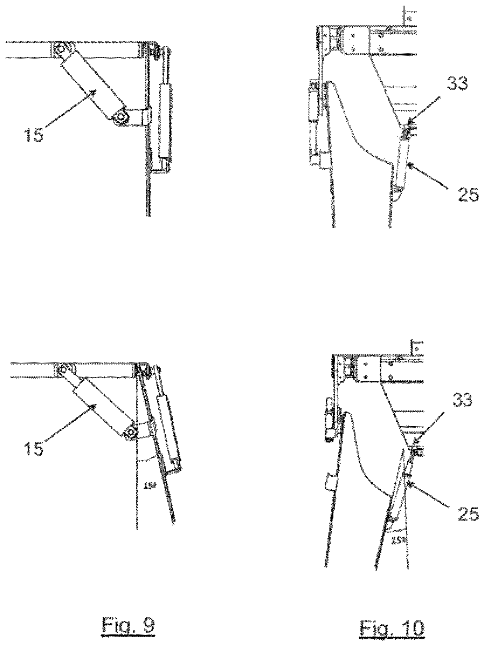

Figure 9 shows an exoskeleton module with a hip sensor illustrated in a non-abducted and an abducted state of 15°.
Figure 10 shows an exoskeleton module with a hip actuator in an abducted and a non-abducted state.

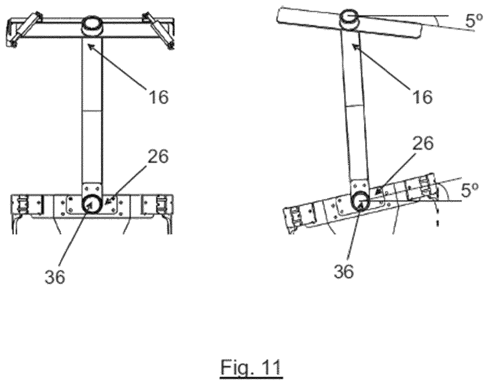

Figure 11 shows an exoskeleton module with a sensor in the upper part of the spine and an actuator in lower part of the spine, illustrated without and with swinging of 10°.

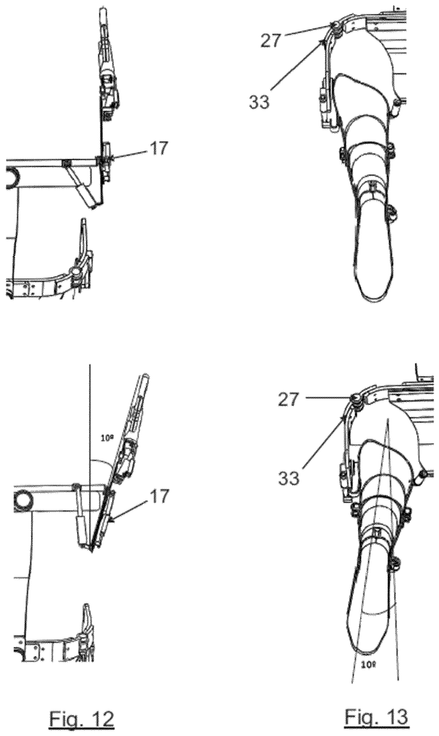

Figure 12 shows an exoskeleton module with a shoulder sensor without and with rotation of 10°.
Figure 13 shows an exoskeleton module with a hip actuator without and with rotation of 10°.

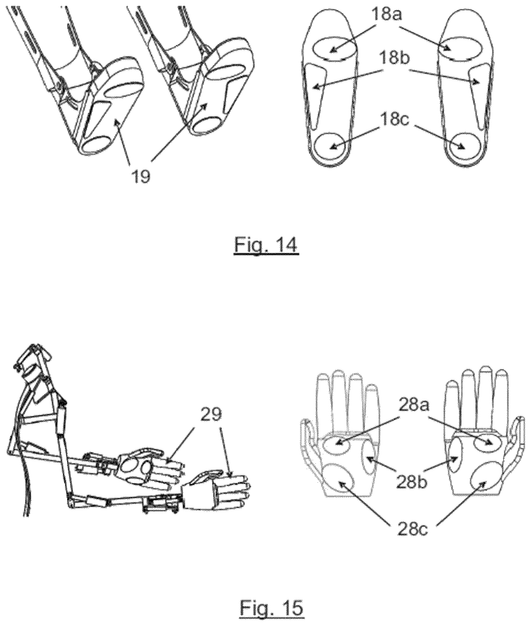

Figure 14 shows an exoskeleton module with sensors in the sole of the foot.
Figure 15 shows an exoskeleton module with actuators in the palms of the hands.

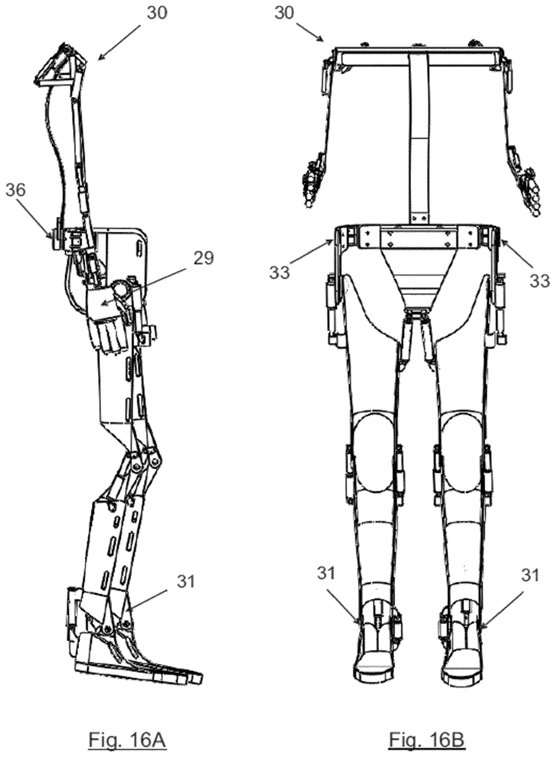

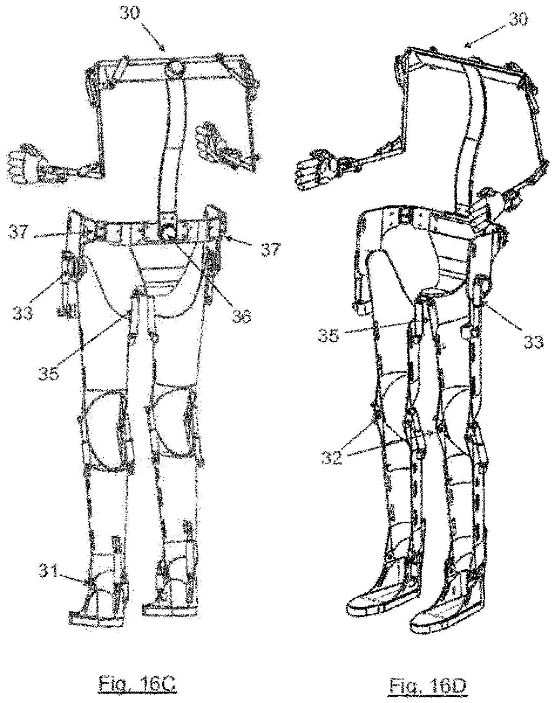

Figure 16 shows complete side, front, and perspective views of the exoskeleton.

### DETAILED DESCRIPTION OF THE INVENTION

A non-limiting embodiment is illustrated in reference to the drawings. The embodiment provides for a suitable gait in patients with paraplegia/paraparesia.

Two parts have been developed to restore this functional ability:
1. One part consists of a metallic mechanical structure that performs each of the movements required for the affected subject to stand and walk in a natural and controlled manner at all times in real time. To that end, an exoskeleton 30 has been developed, consisting of the following elements (exo-foot, exo-tibia, exo-femur, exo-hip, and exo-spine) attached by three articulations on each side and a central articulation (exo-spine):
   - Exo-ankle articulation 31: attachment of the exo-foot with the exo-tibia. This articulation consists of two actuators that allows the flexion-extension and abduction-adduction movements of the ankle, both controlled by two sensors located in the wrist.
   - Exo-knee articulation 32: attachment of the exo-tibia with the exo-femur. This articulation allows the flexion-extension movement through an actuator controlled by a sensor in the elbow.
   - Exo-hip articulation 33: attachment of the exo-femur and the exo-hip. This articulation consists of three movements (flexion-extension, abduction-adduction, and rotation), so three actuators are required on each side. The flexion-extension movement is performed through two actuators located between the exo-femur and the exo-hip in the front part. The abduction-adduction movement is performed through two actuators located on the inner faces of the hip in the attachment of the exo-hip with the exo-femur; the sensors that move these actuators are located in the upper part of the shoulders. The rotational movement is performed through two actuators located on the outer faces of the hip; the respective sensors are also located in the upper part of the shoulder.
   - Exo-spine articulation 34: attachment of the exo-hip and the exo-spine. It provides for the swinging movement through an actuator located between the rear part of the exo-hip and the exo-spine, since this movement can be performed as a result of the swinging of the pelvis. The sensor that makes this movement possible is located in the cervical-thoracic region.
2. Another part consists of the development of an outsole 19 for supporting the exoskeleton 30 on the ground in order to maintain the necessary balance of the subject, said outsole 19 consisting of several cavities required to transmit the different pressures received by the outsole 19 to a sensory region of the subject, such as the hand. Each of these cavities has an associated pressure sensor 18a, 18b, 18c, one being for the front region of the foot 18a, another one being for the outer lateral region 18b, and a third one being for the region of the heel 18c. Said regions of the foot are connected through a glove 29 with the front region 28a of the palm of the hand (proximal palmar sinus), lateral region 28b (hypothenar eminence and distal palmar sinus) and region of the distal sinus 28c of the wrist.

The transmission of pressure is directly proportional; a glove 29 with the same cavities is used to receive pressure states at all times and to enable correct the position in a natural manner, as if the user actually has his or her foot on the ground.

### Degrees of mobility

Wrist - Ankle: The flexion-extension movement of the ankle is controlled by a first wrist sensor 11, which moves an ankle actuator 21 located in the rear part of the Achilles tendon. Upon acquiring a certain degree of flexion in the wrist (considering that the initial state corresponds to placing the palm of the hand parallel to the forearm), this same degree of flexion will be transmitted to the ankle (based on the fact that the initial state corresponds to placing the sole of the foot in the horizontal position).

The abduction-adduction movement of the ankle is also controlled by a second wrist sensor 12, which moves a second ankle actuator 22 located on the outer side of the ankle. Rotation of the wrist allows this movement.

Elbow - Knee: The flexion-extension movement of the knee is controlled by an elbow sensor 12, which moves a knee actuator 22 located on the outer part of the knee, and another one located on the inner part of the knee (the movement of which being identical and simultaneous). When a certain degree of flexion is acquired in the elbow (based on the fact that the initial state is equivalent to placing the arm in the extended position), this same degree of flexion will be transmitted to the knee (considering that the initial state corresponds to keeping the leg extended).

Shoulder - Hip: The flexion-extension movement of the hip is controlled by a shoulder sensor 13, which moves a second hip actuator 33. When a certain degree of flexion is acquired in the shoulder (considering that the initial state corresponds to placing the arm in the vertical position, i.e., placed against the body), this same degree of flexion is transmitted to the hip (the initial state of which corresponds to placing the leg in the vertical position).

The abduction-adduction movement of the hip is controlled by a third shoulder sensor 17 located on the shoulder, which moves a third hip actuator 27 located on the inner part of the hip. When a certain degree of abduction-adduction is acquired in the shoulder (considering that the initial state corresponds to placing the arm in the vertical position, placed against the body), this same degree of abduction-adduction is transmitted to the hip (the initial state of which corresponds to keeping the leg straight, with the sole of the foot on the ground).

The rotational movement of the hip is controlled by a shoulder sensor 13 located on the shoulder, which moves a first hip actuator 23 located in the outer part of the hip. When a certain degree of rotation is acquired in the shoulder (considering that the initial state corresponds to keeping the elbow towards the rear part), this same degree of rotation is transmitted to the hip (the initial state of which corresponds to keeping the toes facing straight forward).

Spine - Hip: The swinging movement of the hip is controlled by a spine sensor 16 located in the cervical-thoracic region, which moves a second hip actuator 26 located in the lower part of the spine, in the articulation attaching the exo-spine with the exo-hip. When a certain degree of swinging is acquired in the upper part of the back-shoulders (considering that the initial state corresponds to keeping the shoulders at the same height), this degree of swinging is transmitted inversely to the exo-hip (considering that the initial state corresponds to keeping the exo-hip and the exo-spine perpendicular to one another).

### Types of sensors and actuators used

The types of sensors used in the exoskeleton are rotary- and linear-type encoders, pressure sensors - fluid chambers - air chambers, gyroscopes, accelerometers.

The types of actuators used are motors, (rotary and linear) servomotors, and vibration motors.

### Processing means

Built-in electronic systems are preferably used for processing the information. These processing means are designed in a dedicated and specific manner to manage operation of the devices described above. Use of general-purpose operating systems that slow down movement generation is thereby avoided.

There are two levels of actuation of these processing means:
The first level is when the user is using the exoskeleton to walk; this requires minimal signal processing to obtain the quickest results, since the response given by the exoskeleton to the user's movements must be instantaneous, i.e., at the same time that the user moves a body part, which acts like a signal emitter, the corresponding part of the exoskeleton moves with the degrees and quickness indicated by the user. At this level, movements are not pre-programmed, i.e., the user has the freedom to decide how each part of the exoskeleton is moved in real time, where he or she can create his or her own way of walking. Nevertheless, there are safety limits in place to protect the user from movements that may injure him or her.

The second level is activated when the user stops in the upright position and disconnects the movement of the exoskeleton to enable using his or her arms freely. In this case, the processing means handle the balance of the exoskeleton, so it requires a more complex signal processing and pre-programmed movements; in this case, the exoskeleton does not have to walk, but rather simply remain upright and perform those movements to compensate for the shifting of weight generated by the upper part of the user.

Although an embodiment in which the measured angle of flexion is the same as that which the corresponding actuator applies to the articulated structure of the exoskeleton has been illustrated in the drawings, other configurations in which the correspondence is more complex are possible.

### REFERENCE NUMBERS

11 First wrist sensor
21 First ankle actuator
12 Elbow sensor
22 Knee actuator
13 First shoulder sensor
23 First hip actuator
14 Second wrist sensor
24 Second ankle actuator
15 Second shoulder sensor
25 Second hip actuator
16 Spine sensor
26 First hip actuator
17 Third shoulder sensor
27 Second hip actuator
18a, 18b, 18c Outsole pressure sensors
28a, 28b, 28c Hand pressure transducers
19 Outsole
29 Glove
30 Exoskeleton
31 Exo-ankle articulation
32 Exo-knee articulation
33 Exo-hip articulation
34 Exo-spine articulation

## Claims

1. System to assist a person in walking, wherein said system comprises an exoskeleton (30) to be fitted to a person, wherein the exoskeleton (30) comprises the following articulated structures (31-33), configured for applying movements in the person's lower extremity:
- an exo-ankle articulation (31) comprising an attachment of an exo-foot with an exo-tibia, comprising two actuators (21, 24) configured to allow flexion-extension and abduction-adduction movements of the person's ankle, said actuators (21, 24) being connected to two sensors (11, 14) located in the wrist;
- an exo-knee articulation (32) comprising an attachment of the exo-tibia with an exo-femur, configured to allow flexion-extension movement of the person's knee, through an actuator (22) connected to a sensor (12) in the elbow; wherein the exoskeleton (30) further comprises:
- an exo-hip articulation (33) attached to the exo-femur, configured to allow flexion-extension, abduction-adduction and rotation movements of the person's hip through a first, second and third actuators (23, 25, 27), wherein the flexion-extension movement is performed through the first actuator (23) located between the exo-femur and the exo-hip in the front part of the person's hip, the abduction-adduction movement is performed through the second actuator (25) located on the inner face of the person's hip in an attachment of the exo-hip with the exo-femur, and the rotation movements are performed through the third actuator (27) located on the outer face of the person's hip, and wherein the three actuators (23, 25, 27) are connected to a first, second and third sensors (13, 15, 17) located in the upper part of the shoulders;
wherein the system further comprises processing means configured for receiving information from the sensors (11-15, 17) about the movements of the person's wrist, elbow and shoulders, and for translating said movements into an actuation of their corresponding actuators (21-25, 27), thereby controlling the movement of the articulations (31-33) in real time.

2. System according to claim 1, **characterized in that** the system comprises an exo-spine (34) articulation, further comprising an attachment of the exo-hip with an exo-spine, configured to allow a swinging movement of the person's pelvis through an actuator (26) located between a rear part of the exo-hip and the exo-spine, wherein:
- said actuator (26) is connected to a sensor (16) located in the cervical-thoracic region, and
- the processing means is configured for receiving information from the cervical-thoracic sensor (16) about the swinging movement of the person's pelvis, and for translating said movement into an actuation of the corresponding actuator (26), thereby controlling the movement of the exo-spine articulation (34).

3. System according to any of the preceding claims, **characterized in that** the corresponding sensors (11-15, 17) are adapted for measuring the flexion speed of the wrist, the elbow and the shoulder measured by the sensors (11-15, 17), and **in that** the processing means is configured for translating said flexion speed into the flexion speed applied by the corresponding actuator (21-25, 27) in the corresponding articulations (31-33).

4. System according to claim to any of the preceding claims, **characterized in that** the system comprises an outsole (19) for supporting the exoskeleton (30) on the ground, wherein the outsole (19) houses pressure sensors (18a, 18b, 18c) configured for measuring the pressure from the person's foot received by the outsole (19).

5. System according to claim 4, **characterized in that** the pressure sensors (18a, 18b, 18c) are configured for transmitting the measured pressure on the person's hand, by means of corresponding transducers (28a, 28b, 28c).

6. System according to claim 5, **characterized in that** the pressure sensors (18a, 18b, 18c) are adapted for measuring the pressure at the front region of the foot, at the outer lateral region of the foot and at the heel region of the foot.

7. System according to claim 6, **characterized in that** the system comprises a glove (29) housing the transducers (28a, 28b, 28c), and wherein said transducers (28a, 28b, 28c) are adapted for transmitting the pressures measured at the front region of the foot, the outer lateral region of the foot and the heel region of the foot to the front region of the palm of the hand, the lateral region of the palm of the hand and the region of the distal sinus of the wrist, respectively.

8. System according to any of the preceding claims, **characterized in that,** for walking, the processing means is configured for receiving information from the sensors (11-15, 17) about the flexion movements of the person's wrist, elbow and shoulders, and for translating said movements into a proportional actuation of their corresponding actuators (21-25, 27).

## Patentansprüche

1. System zur Unterstützung eines Menschen beim Gehen, wobei das genannte System ein Exoskelett (30) umfasst, um es auf einem Menschen aufgebracht zu werden, wobei das Exoskelett (30) die folgenden gelenkigen Strukturen (31-33) umfasst, welche dazu ausgebildet sind, Bewegungen in der unteren Extremität des Menschen anzuwenden;
- ein Exoknöchel-Gelenk (31), welches einen Anschluss eines Exofußes mit einem Exoschienbein umfasst, umfassend zwei Stellglieder (21, 24) welche dazu ausgebildet sind, Beugungs-Streckungs- und Abduktions-Adduktions-Bewegungen des Knöchels des Menschen zu erlauben, wobei die genannten Stellglieder (21, 24) mit zwei Sensoren (11, 14) verbunden sind, welche sich im Handgelenk befinden;
- ein Exoknie-Gelenk (32), welches einen Anschluss des Exoschienbeins mit einem Exofemur umfasst, welches dazu ausgebildet ist, die Beugungs-Streckungs-Bewegung des Knies des Menschen zu erlauben, durch ein Stellglied (22), welches mit einem Sensor (12) im Ellbogen verbunden ist;
wobei das Exoskelett (30) zusätzlich Folgendes umfasst:
- ein Exohüfte-Gelenk (33), welches am Exofemur angeschlossen ist, welches dazu ausgebildet ist, Beugungs-Streckungs-, Abduktions-Adduktions- und Rotationsbewegungen der Hüfte des Menschen durch ein erstes, zweites und drittes Stellglieder (23, 25, 27) zu erlauben, wobei die Beugungs-Streckungs-Bewegung durch das erste Stellglied (23) durchgeführt wird, welches sich zwischen dem Exofemur und der Exohüfte im Vorderteil der Hüfte des Menschen befindet, die Abduktions-Adduktions-Bewegung durch das zweite Stellglied (25) durchgeführt wird, welches sich auf der Innenfläche der Hüfte des Menschen in einem Anschluss der Exohüfte mit dem Exofemur befindet, und die Rotationsbewegungen durch das dritte Stellglied (27) durchgeführt wird, welches sich auf der Außenfläche der Hüfte des Menschen befindet, und wobei die drei Stellglieder (23, 25, 27) mit einem ersten, zweiten und dritten Sensor (13, 15, 17) verbunden sind, welches sich im oberen Teil der Schultern befinden;
wobei das System zusätzlich Verarbeitungsmittel umfasst, welche dazu ausgebildet sind, Information aus den Sensoren (11-15, 17) über die Bewegungen des Handgelenks, des Ellbogens und der Schultern des Menschen zu empfangen, und die genannten Bewegungen in eine Betätigung deren entsprechenden Stellglieder (21-25, 27) umzuwandeln, wodurch die Bewegung der Gelenke (31-33) in Echtzeit kontrolliert wird.

2. System nach Anspruch 1, **dadurch gekennzeichnet, dass** das System ein Exowirbelsäule-Gelenk (34) umfasst, zusätzlich umfassend einen Anschluss der Exohüfte mit einer Exowirbelsäule, welcher dazu ausgebildet ist, eine schwingende Bewegung des Beckens des Menschen durch ein Stellglied (26) zu erlauben, welches sich zwischen einem Hinterteil der Exohüfte und der Exowirbelsäule befindet, wobei:
- das genannte Stellglied (26) mit einem Sensor (16) verbunden ist, welcher sich im Nacken-BrustBereich befindet, und
- die Verarbeitungsmittel dazu ausgebildet sind, Information aus dem Nacken-Brust Sensor (16) über die schwingende Bewegung des Beckens des Menschen zu empfangen, und die genannte Bewegung in eine Betätigung des entsprechenden Stellgliedes (26) umzuwandeln, wodurch die Bewegung des Exowirbelsäule-Gelenks (34) kontrolliert wird.

3. System nach einem der vorhergehenden Ansprüche, **dadurch gekennzeichnet, dass** die entsprechenden Sensoren (11-15, 17) dazu angepasst sind, die von den Sensoren (11-15, 17) gemessene Beugungsgeschwindigkeit des Handgelenks, des Ellbogens und der Schulter zu messen, und dass die Verarbeitungsmittel dazu ausgebildet sind, die genannte Beugungsgeschwindigkeit in die Beugungsgeschwindigkeit umzuwandeln, welche vom entsprechenden Stellglied (21-25, 27) in den entsprechenden Gelenken (31-33) angewendet wird.

4. System nach einem der vorhergehenden Ansprüche, **dadurch gekennzeichnet, dass** das System eine Laufsohle (19) zur Stützung des Exoskeletts (30) auf dem Boden umfasst, wobei die Laufsohle (19) Drucksensoren (18a, 18b, 18c) aufnimmt, welche dazu ausgebildet sind, den aus der Laufsohle (19) empfangenen Druck vom Fuß des Menschen zu messen.

5. System nach Anspruch 4, **dadurch gekennzeichnet, dass** die Drucksensoren (18a, 18b, 18c) dazu ausgebildet sind, den auf der Hand des Menschen gemessenen Druck mittels entsprechender Umformer (28a, 28b, 28c) zu übertragen.

6. System nach Anspruch 5, **dadurch gekennzeichnet, dass** die Drucksensoren (18a, 18b, 18c) dazu angepasst sind, den Druck am Vorderbereich des Fußes, am äußeren Seitenbereich des Fußes und am Fersenbereich des Fußes zu messen.

7. System nach Anspruch 6, **dadurch gekennzeichnet, dass** das System einen Handschuh (29) umfasst, welcher die Umformer (28a, 28b, 28c) aufnimmt, und wobei die genannten Umformer (28a, 28b, 28c) dazu angepasst sind, jeweils die am Vorderbereich des Fußes, am äußeren Seitenbereich des Fußes und am Fersenbereich des Fußes zum Vorderbereich der Handfläche, zum Seitenbereich der Handfläche und zum Bereich des distalen Sinus des Handgelenks gemessenen Drücke zu übertragen.

8. System nach einem der vorhergehenden Ansprüche, **dadurch gekennzeichnet, dass**, zum Gehen, die Verarbeitungsmittel dazu ausgebildet sind, Information aus den Sensoren (11-15, 17) über die Beugungsbewegungen des Handgelenks, des Ellbogens und der Schultern des Menschen zu empfangen, und die genannten Bewegungen in eine proportionale Betätigung deren entsprechenden Stellglieder (21-25, 27) umzuwandeln.

## Revendications

1. Système pour aider une personne à marcher, dans lequel ledit système comprend un exosquelette (30) à monter sur une personne, dans lequel l'exosquelette (30) comprend les structures articulées suivantes (31-33), configurées pour appliquer des mouvements à l'extrémité inférieure de la personne :
- une articulation d'exo-cheville (31) comprenant une attache d'un exo-pied avec un exo-tibia, comprenant deux actionneurs (21, 24) configurés pour permettre des mouvements de flexion-extension et d'abduction-adduction de la cheville de la personne, lesdits actionneurs (21, 24) étant reliés à deux capteurs (11, 14) situés sur le poignet ;
- une articulation d'exo-genou (32) comprenant une attache de l'exo-tibia avec un exo-fémur, configuré pour permettre un mouvement de flexion-extension du genou de la personne, par le biais d'un actionneur (22) relié à un capteur (12) dans le coude,
dans lequel l'exosquelette (30) comprend en outre :
- une articulation d'exo-hanche (33) fixée à l'exo-fémur, configurée pour permettre des mouvements de flexion-extension, d'abduction-adduction et de rotation de la hanche de la personne par le biais d'un premier, un second et un troisième actionneurs (23, 25, 25, 27), dans lequel le mouvement de flexion-extension est réalisé par le biais du premier actionneur (23) situé entre l'exo-fémur et l'exo-hanche dans la partie avant de la hanche de la personne, le mouvement d'abduction-adduction est réalisé par le biais du second actionneur (25) situé sur la face interne de la hanche de la personne dans une attache de l'exo-hanche avec l'exo-fémur, et les mouvements de rotation sont réalisés par le biais du troisième actionneur (27) situé sur la face externe de la hanche de la personne, et dans lequel les trois actionneurs (23, 25, 27) sont reliés au premier, second et troisième capteurs (13, 15, 17) situés sur la partie supérieure des épaules ;
dans lequel le système comprend en outre des moyens de traitement configurés pour recevoir de l'information à partir des capteurs (11-15, 17) à propos des mouvements du poignet, du coude et des épaules de la personne, et pour traduire lesdits mouvements en un actionnement de leurs actionneurs correspondants (21-25, 27), en contrôlant de la sorte le mouvement des articulations (31-33) en temps réel.

2. Système selon la revendication 1, **caractérisé en ce que** le système comprend une articulation d'exo-colonne vertébrale (34), comprenant en outre une attache de l'exo-hanche avec une exo-colonne vertébrale, configurée pour permettre un mouvement oscillant du pelvis de la personne par le biais d'un actionneur (26) situé entre une partie arrière de l'exo-hanche et l'exo-colonne vertébrale,
dans lequel :
- ledit actionneur (26) est relié à un capteur (16) situé dans la région cervicale-thoracique, et
- les moyens de traitement sont configurés pour recevoir de l'information à partir du capteur cervical-thoracique (16) à propos du mouvement oscillant du pelvis de la personne, et pour traduire ledit mouvement en un actionnement de l'actionneur correspondant (26), en contrôlant de la sorte le mouvement de l'articulation d'exo-colonne vertébrale (34).

3. Système selon l'une quelconque des revendications précédentes, **caractérisé en ce que** les capteurs correspondants (11-15, 17) sont aptes à mesurer la vitesse de flexion du poignet, du coude et de l'épaule mesurée par les capteurs (11-15, 17), et **en ce que** les moyens de traitement sont configurés pour traduire ladite vitesse de flexion en la vitesse de flexion appliquée par l'actionneur correspondant (21-25, 27) dans les articulations correspondantes (31-33).

4. Système selon l'une quelconque des revendications précédentes, **caractérisé en ce que** le système comprend une semelle extérieure (19) pour supporter l'exosquelette (30) sur terre dans lequel la semelle extérieure (19) loge des capteur de pression (18a, 18b, 18c) configurés pour mesurer la pression du pied de la personne reçu par la semelle extérieure (19).

5. Système selon la revendication 4, **caractérisé en ce que** les capteurs de pression (18a, 18b, 18c) sont configurés pour transmettre la pression mesurée sur la main de la personne, par le biais des transducteurs correspondants (28a, 28b, 28c).

6. Système selon la revendication 5, **caractérisé en ce que** les capteurs de pression (18a, 18b, 18c) sont aptes à mesurer la pression dans la région avant du pied, la région latérale extérieure du pied et la région de talon du pied.

7. Système selon la revendication 6, **caractérisé en ce que** le système comprend un gant (29) logeant les transducteurs (28a, 28b, 28c), et dans lequel lesdits transducteurs (28a, 28b, 28c) sont aptes à transmettre les pressions mesurées sur la région avant du pied, la région latérale extérieure du pied et la région de talon du pied à la région avant de la paume de la main, la région latérale de la paume de la main et la région du sinus distal du poignet, respectivement.

8. Système selon l'une quelconque des revendications précédentes, **caractérisé en ce que**, pour marcher, les moyens de traitement sont configurés pour recevoir de l'information à partir des capteurs (11-15, 17) à propos des mouvements de flexion du poignet, du coude et des épaules de la personne, et pour traduire lesdits mouvements en un actionnement proportionnel de leurs actionneurs correspondants (21-25, 27).
